# EUROPEAN PATENT SPECIFICATION

(11) **EP 2 745 786 B1**
(45) Date of publication and mention of the grant of the patent: **30.08.2017**
(21) Application number: 13198276.1
(22) Date of filing: 19.12.2013
(51) Int. Cl.: A61B 17/17

(54) **Trajectory guide**
Bahnführung
Guide de trajectoire

(30) Priority: 21.12.2012 US 201261740873 P; 04.12.2013 US 201314096895
(43) Date of publication of application: 25.06.2014
(73) Proprietor: Wright Medical Technology, Inc., Memphis, TN 38117 (US)
(72) Inventor: Thoren, Brian, Memphis, Tennessee 38119 (US); Reed, Wesley, Libertyville, Illinois 60048 (US); Cramer, Thomas, Gainesville, Florida 32605 (US); Lowery, Gary, Eads, Tennessee 38028 (US); Cummings, Shannon, Hernando, Mississippi 38632 (US)
(74) Representative: Gerbino, Angelo

(56) References cited:
- WO-A1-2006/081483
- US-A1- 2007 173 843
- US-A1- 2008 188 852
- US-A1- 2009 088 767

## Description

### Field of invention

The present invention relates to devices for positioning screws or k-wires during surgical procedures, and more particularly to a trajectory guide that can be used with existing drill guides or the like to direct the path along which a screw or k-wire enters a bone.

More specifically the present invention relates to a device having the features indicated in the preamble of claim 1 which follows. A known device of this kind is disclosed in US 2008/188 852.

### Background of the invention

The field of orthopaedic medicine has grown tremendously in the past fifty years as surgical techniques, implants and instrumentation have improved. Small bone that are often located in the extremities such as the hands and feet, are frequently subject to the need for re-constructive surgery for example, as a result of trauma, to counteract the effects of aging or to repair congenital deformities. Such surgical techniques often involve the fixing of the bones with an orthopaedic plate and compression screws.

For example, during a typical procedure on the foot, a compression screw will be placed on the underside (e.g., plantar) of an orthopaedic plate during the surgery. During this procedure, screws that have already been positioned in the plate must not be engaged by the newly introduced screw. In many instances the surgeon may only estimate the appropriate trajectory of the screw, sometimes leading to problems. Cannulated guides are often used during this procedure. The positions of previously inserted screws or k-wires are often difficult for the surgeon to ascertain. This may lead to improper positioning or an unwanted interaction between an already inserted screw and a next screw to be inserted during the procedure. Known guides often lack the accuracy and precision necessary for determining the trajectory of .screws as they are placed through a bone.

Accordingly, it is desirable to design a guide for controlling the trajectory of screws and k-wires relative to an orthopaedic plate so that securing screws may converge in order to cause compression or increase the pull out strength, while minimizing the occurrence of a screw impinging upon or conflicting with the desired placement of another screw.

### Summary of invention

According to the invention, a guide having the features disclosed in claim 1 which follows is provided for determining the trajectory of a compression screw relative to the location at which a screw will be located within an orthopaedic plate. The guide includes a first drill guide adapted to be releasably coupled to the orthopaedic plate into which one or more screws will be set. A first arm adapted to be coupled to the first drill guide. A second arm is provided that includes a distal end coupled to the first arm and a proximal end defining a pair of openings, e.g., slots or bores. The pair of openings are each adapted to releasably position a second drill guide in spaced-apart relation to the drill guide engaging the orthopaedic plate such that the second guide defines a trajectory for a compression screw that is nonintersecting with the trajectory that will be followed by screws that are eventually set in the orthopaedic plate.

### Brief description of the drawings

These and other features and advantages of the present invention will be more fully disclosed in, or rendered obvious by, the following detailed description of the preferred embodiment of the invention, which is to be considered together with the accompanying drawings wherein like numbers refer to like parts and further wherein:
FIG. 1 is a perspective view of a trajectory guide, bone plat and drill guide formed in accordance with the present invention;
FIG. 2 is a further perspective view of the trajectory guide, bone plate, and drill guide shown in FIG. 1;
FIG. 3 is an exploded perspective view of the trajectory guide, bone plate and drill guide shown in FIGS. 1 and 2;
FIG. 4 is a perspective view of a long arm formed in accordance with one embodiment of the invention
FIG. 5 is a side elevational view of a trajectory guide with a drill guide and slotted short arm in accordance with one embodiment of the invention;
FIG. 6 is a partially broken away perspective view of a trajectory guide, bone plate, and drill guide depicted during a portion of a surgical procedure on a foot;
FIG. 7 is a partially broken away perspective view of another stage of the surgical procedure shown in Fig. 6;
FIG. 7a is a partially broken away perspective view, similar to that shown in Fig. 7, but at a later stage of the surgical procedure, illustrating a compression screw being inserted on a plantar side of a patient's foot along a pre-defined trajectory resulting from use of the invention;
FIG. 7b is a partially broken away perspective view, similar to that shown in Fig. 7a, at a later stage of the surgical procedure, illustrating a cannulated compression screw being inserted on a plantar side of a patient's foot along a pre-defined trajectory resulting from use of the invention with a k-wire;
FIG. 8 is a perspective view of an alternative embodiment of trajectory guide assembled with a drill guide and bone plate in accordance with the invention;
FIG. 9 is a perspective view of an alternative embodiment trajectory guide;
FIG. 10 is an exploded perspective view of the trajectory guide shown in FIG. 9; and
FIG. 11 is a broken away perspective view of an alternative embodiment of trajectory guide combined with a bone plate and drill guide depicted during a portion of surgery on a human foot.

### Detailed description of the preferred embodiments

This description of preferred embodiments is intended to be read in connection with the accompanying drawings, which are to be considered part of the entire written description of this invention. The drawing figures are not necessarily to scale and certain features of the invention may be shown exaggerated in scale or in somewhat schematic form in the interest of clarity and conciseness. In the description, relative terms such as "horizontal," "vertical," "up," "down," "top" and "bottom" as well as derivatives thereof (e.g., "horizontally," "downwardly," "upwardly," etc.) should be construed to refer to the orientation as then described or as shown in the drawing figure under discussion. These relative terms are for convenience of description and normally are not intended to require a particular orientation. Terms including "inwardly" versus "outwardly," "longitudinal" versus "lateral" and the like are to be interpreted relative to one another or relative to an axis of elongation, or an axis or center of rotation, as appropriate. Terms concerning attachments, coupling and the like, such as "connected" and "interconnected," refer to a relationship wherein structures are secured or attached to one another either directly or indirectly through intervening structures, as well as both movable or rigid attachments or relationships, unless expressly described otherwise. The term "operatively connected" is such an attachment, coupling or connection that allows the pertinent structures to operate as intended by virtue of that relationship. In the claims, means-plus-function clauses, if used, are intended to cover the structures described, suggested, or rendered obvious by the written description or drawings for performing the recited function, including not only structural equivalents but also equivalent structures. To the extent that the term "includes" or "including" is employed in the detailed description or the claims, it is intended to be inclusive in a manner similar to the term "comprising" as that term is interpreted when employed as a transitional word in a claim. Furthermore, to the extent that the term "or" is employed in the detailed description or claims (e.g., A or B) it is intended to mean "A or B or both." When the applicants intend to indicate "only A or B but not both" then the term "only A or B but not both" will be employed. Thus, use of the term "or" herein is the inclusive, and not the exclusive use. See, Bryan A. Garner, A Dictionary of Modern Legal Usage 624 (2d. Ed. 1995).

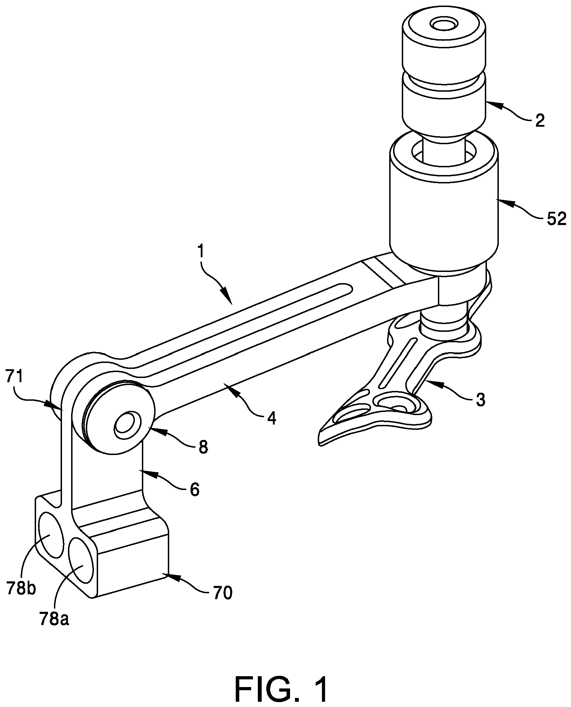

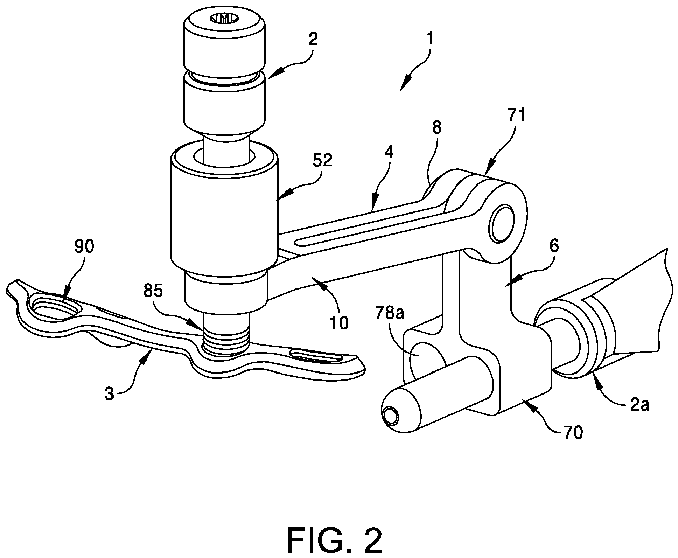

Referring to Figs. 1-5, a trajectory guide 1 is provided that is suitable for orienting a drill guide 2, and thereby the eventual path or trajectory of a compression screw or k-wire as it enters a patient so as to avoid screws or k-wires that are to be set subsequently in an orthopaedic plate 3. In other words, the invention provides the surgeon the ability to guide the trajectory of a screw or k-wire such that it is located in the patient's body in nonintersecting spaced apart relation to objects that are to be positioned in the body at a subsequent step in the surgical procedure.

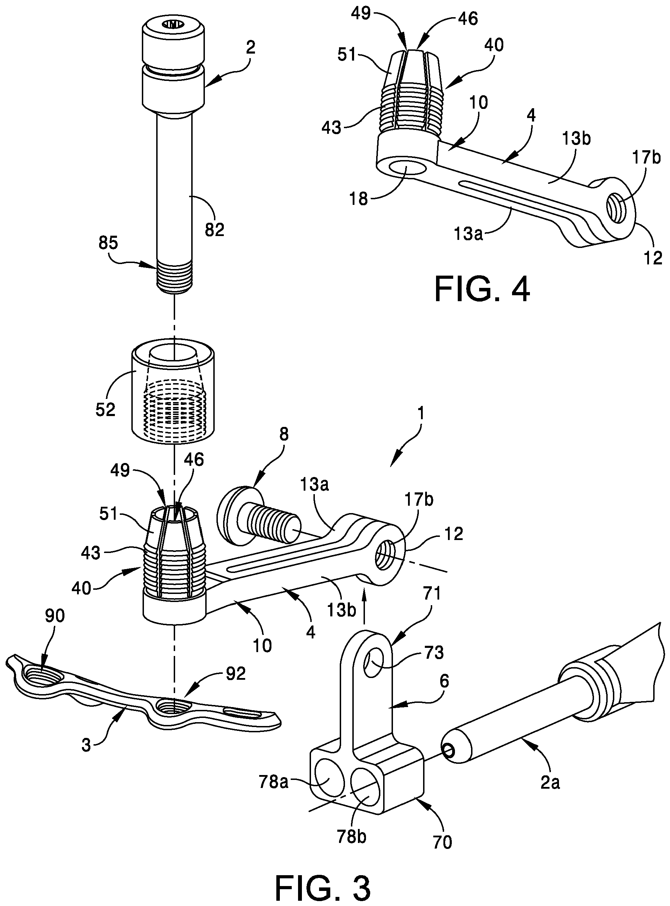

Trajectory guide 1 often includes a long arm 4, a short arm 6 and a thumb screw 8. Long arm 4 includes a distal end 10 and a proximal end 12. A pair of beams 13a, 13b project outwardly from distal end 10 of long arm 4 in substantially parallel, spaced relation to one another. The proximal ends of beams 13a, 13b define confronting, coaxial through bores 17a, 17b. Distal end 10 of long arm 4 defines a through-bore 18 that is often oriented at approximately ninety degrees with respect to coaxial through bores 17a, 17b (Fig. 4).

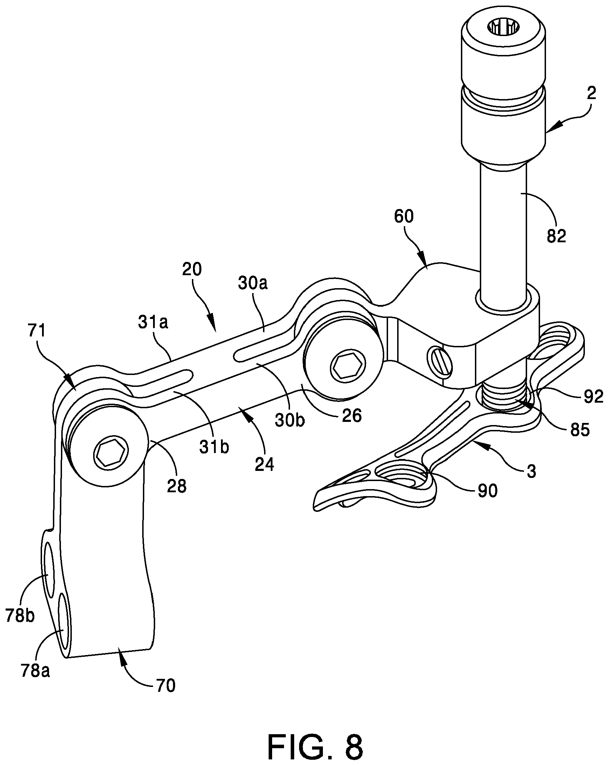

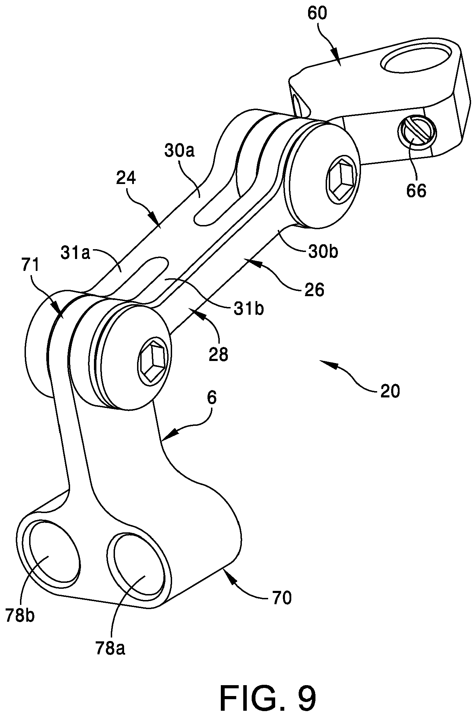

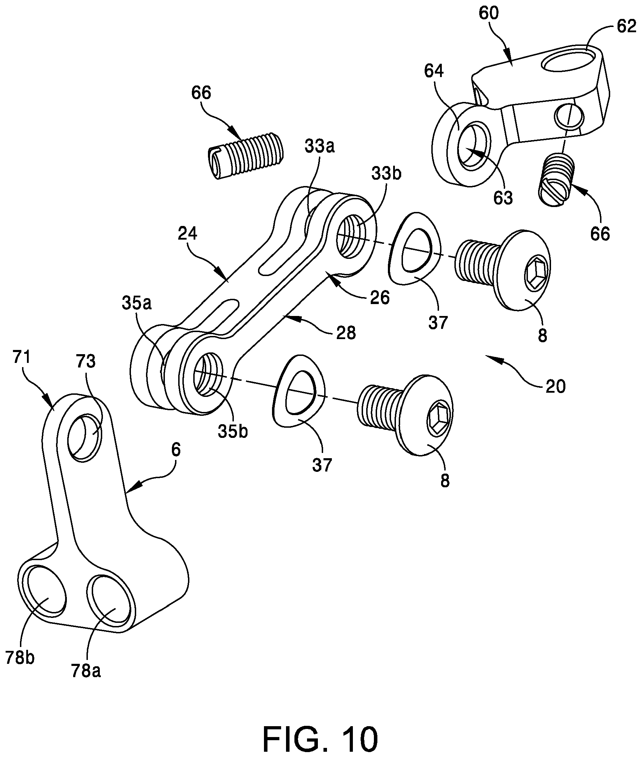

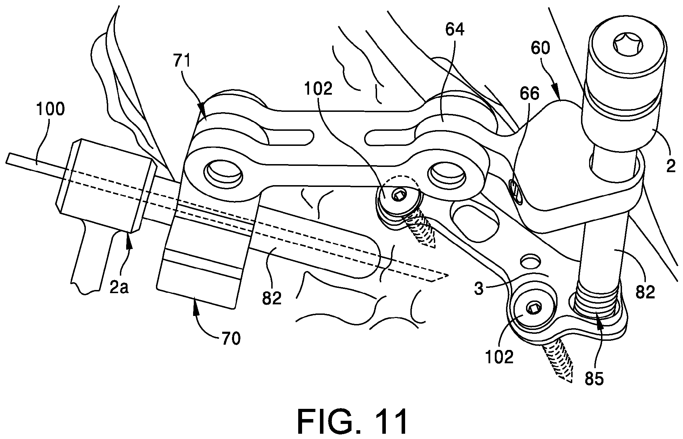

In a multi-degree of freedom embodiment, trajectory guide 20, a long arm 24 includes a distal end 26 and a proximal end 28 (Figs. 8-11). A pair of beams 30a, 30b project outwardly from proximal end 28 of long arm 24 in substantially parallel, spaced relation to one another, and a second pair of beams 31a, 31b project outwardly from distal end 26 of long arm 24 in substantially parallel, spaced relation to one another. The distal ends of beams 30a, 30b define confronting, coaxial through bores 33a, 33b and the proximal ends of beams 31a, 31b define confronting, coaxial through bores 35a, 35b (Fig. 10).

Referring once again to Figs. 1-5, a collet 40 projects upwardly from distal end 10 of long arm 4 (Fig. 3). Collet 40 has a frusto-conical cross-sectional profile, a thread 43 defined about its outer surface, and a through-bore 46. Through bore 46 is coaxially aligned with through-bore 18 defined at distal end 10 of long arm 4. Longitudinally oriented slots 49 divide collet 40 into six arcuate cantilevers 51, that when deflected inwardly, act to grip a shaft located within through bore 46, e.g., a shaft portion of drill guide 2. Often, a collet screw 52 is threaded onto thread 43 of collet 40 to achieve a selective engagement of cantilevers 51 with a circular or rod-like potion of drill guide 2 disposed within through bore 46. In the multi-degree of freedom embodiment of trajectory guide 20, articulating collet 60 includes a guide grip 62 and a pivot plate 64 that projects outwardly from a portion of guide grip 62 (Figs. 8-11). Pivot plate 64 defines a through hole 63 along its length. Guide grip 62 may include releasable gripping features, e.g., ball plungers 66 that allow for releasable gripping of drill guide 2 during operation of the invention.

Referring to Figs. 1-5 and 8-10, short arm 6 includes a distal-block end 70 and a proximal-pivot end 71. Proximal-pivot end 71 of short arm 6 defines a through-bore 73, and distal-block end 70 defines a pair of guide bore openings. In one embodiment, the guide bore openings comprise a pair of slots 75a, 75b arranged in substantially parallel, spaced relation to one another. In another embodiment, the guide bore openings comprise a pair of through-bores 78a, 78b arranged in substantially parallel, spaced relation to one another.

Trajectory guide 1 is assembled prior to shipping as follows. Preliminarily, long arm 4 is assembled to short arm 6 by arranging beams 13a, 13b so that they straddle proximal pivot end 71 of short arm 6. In this position, through-bores 17a, 17b are arranged in aligned coaxial relation with through-bore 73 of short arm 6. Once in this position, thumb screw 8 is inserted through bores 17a, 17b, and 73, one of which bores is threaded, so as to form an articulating joint that pivotally couples long arm 4 to short arm 6. Often a locking, spring washer 37 is placed between thumb screw 8 and long arm 4 so as to ensure that trajectory guide 1 maintains its connectivity through its use, as established by the surgeon.

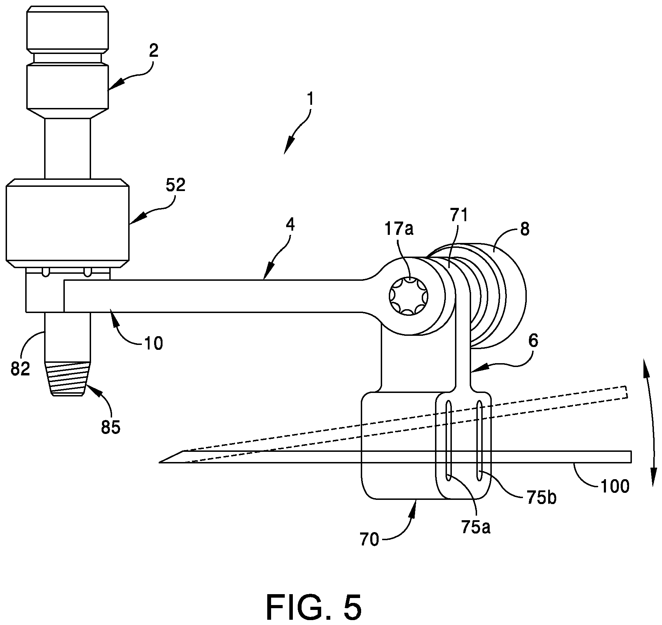

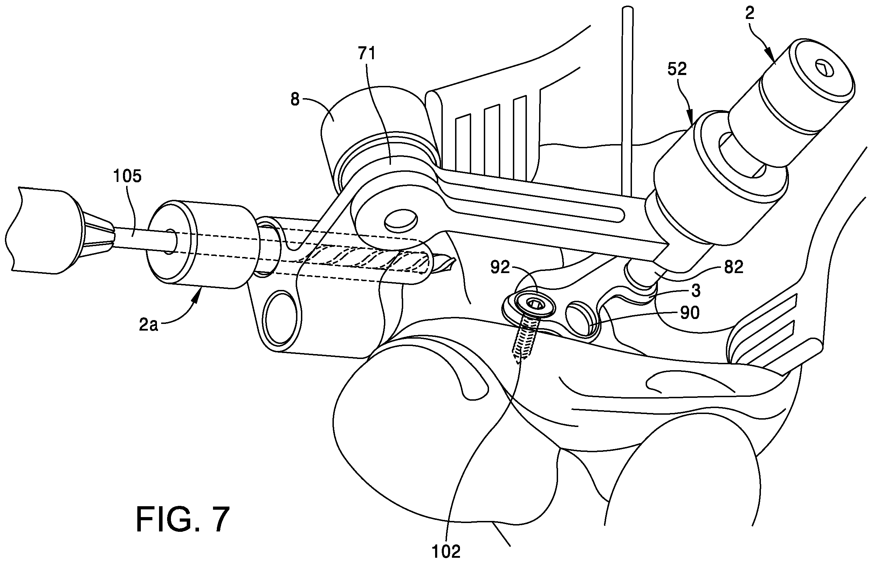

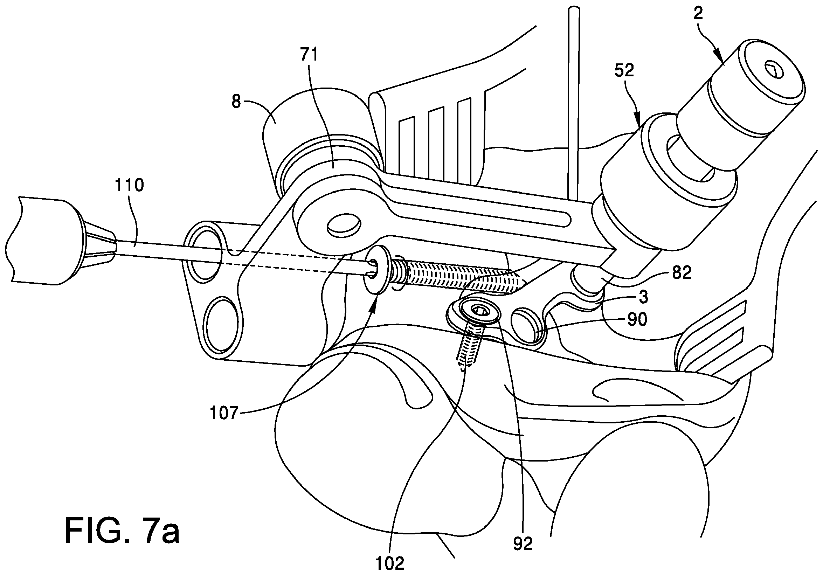

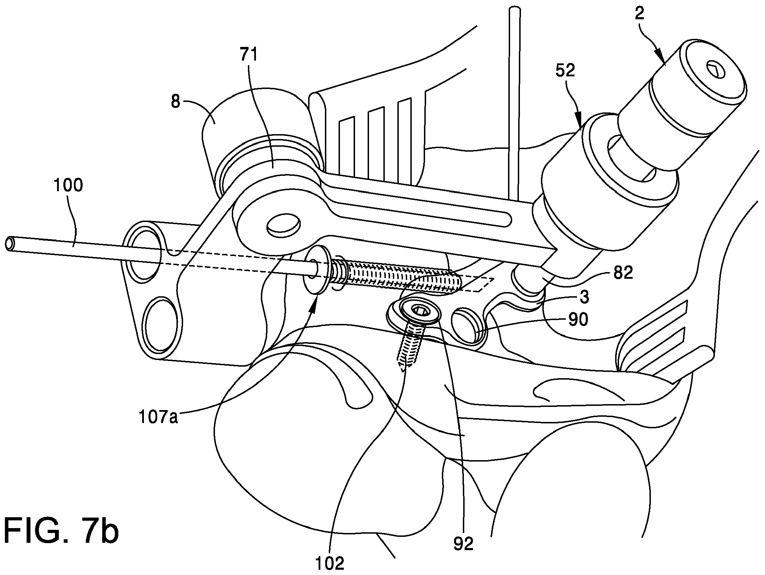

During surgery, a drill guide 2 having a guide cannula 82 may be slidingly located within collet screw 52 and through-bore 46 of collet 40. Once this coupling is complete, trajectory guide 1 is positioned and releasably engaged in orthopaedic plate 3. More particularly, a threaded end 85 of cannula 82 is threaded into corresponding threads 90 located within a target screw hole 92 within orthopaedic plate 3. Once in this position, collet screw 52 is threaded onto thread 43 of collet 40 to achieve a selected engagement of cantilevers 51 about the outer surface of guide cannula 82 as needed. As a result of this arrangement, the central passageway of cannula 82 will be positioned in coaxial alignment with the center of screw hole 92 while, at the same time, being in coplanar parallel relation with short arm 6. Thus, when a k-wire 100 is located in one of pair of slots 75a, 75b (Fig. 5) it will be offset from the plane containing short arm 6 and the central axis of cannula 82. In other words, k-wire 100 will define the eventual path or trajectory of a compression screw 107 or cannulated compression screw 107a, as it enters a patient so as to avoid screws or k-wires that are to be subsequently set along the line of trajectory established by the central passageway of cannula 82 and the center of screw hole 92 of orthopaedic plate 3 (Figs. 7a and 7b).

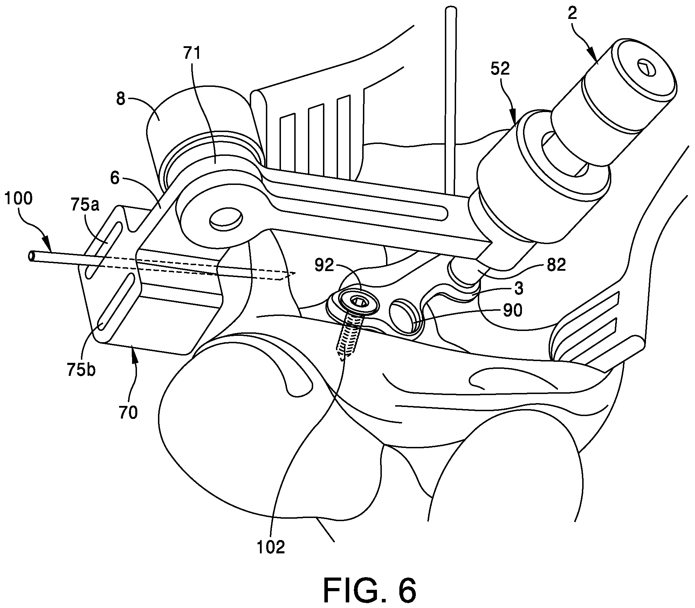

The slotted version of short arm 6 (Fig. 5) may be replaced with a short arm having guide bore openings 78a, 78b by removing thumb screw 8 and switching to the alternate short arm. When a drill guide 2a is then located within one of guide bore openings 78a, 78b arranged in substantially parallel, spaced relation to one another, a drill 105 may be advanced along the line of trajectory previously defined by the positioning of k-wire 100. In many instances this is achieved through use of a cannulated drill 105 so that k-wire 100 is received within a central longitudinal cannula of drill 105 (Fig. 7). As a consequence, a compression screw 107 introduced along the trajectory line originally defined by k-wire 100 (Fig. 6) and now cannula 82 of drill guide 2a, will enter the patient along a line of trajectory that is nonparallel, nonintersecting with the line of trajectory being defined by drill guide 2 when it is assembled to an otherwise empty plate hole of orthopaedic plate 3. In this way, when a screw 102 is positioned within plate hole 92, after drill guide 2 has been removed, screw 102 and compression screw 107 will never meet or cross one another.

Referring to Fig. 7a, trajectory guide 1 is used to place a compression screw 107 on the underside (plantar) of an orthopaedic plate 3 during surgery, while avoiding screws 102 that will be subsequently set in orthopaedic plate 3. When utilizing trajectory guide 20, additional degrees of freedom are provided as a result of articulating collet 60 being pivotally coupled to distal end 26 of long arm 24. In this way, articulating collet 60 may be pivoted about thumb screw 8 located within coaxial through bores 33a, 33b of distal ends of beams 30a, 30b. In some surgical procedures, a cannulated compression screw 107a may be slid over a previously positioned k-wire 100 using trajectory guide 1 or trajectory guide 20 (Fig. 7b).

Thus, by orienting drill guide 2a according to the method of the invention, the trajectory of compression screw 107, as it enters the patient's body, e.g., plantar aspect of a foot, will avoid screws 102 that will be subsequently set in orthopaedic plate 3. In this way, the invention provides the surgeon the ability to guide the trajectory of a compression screw such that it is located in the patient's body in nonintersecting, spaced apart relation to objects that are to be positioned in the body at a subsequent step in the surgical procedure. While the concepts of the present disclosure have been illustrated and described in detail in the drawings and foregoing description, such an illustration and description is to be considered as exemplary and not restrictive in character, it being understood that only illustrative embodiments have been shown and described and that all changes and modifications that come within the scope of the appended claims are desired to be protected.

## Claims

1. A guide for determining the trajectory of a second compression screw relative to a location at which a first compression screw will be subsequently located in an orthopaedic plate (3) comprising:
a first drill guide (2) adapted to be coupled to the orthopaedic plate (3) and defining the trajectory of the first compression screw when coupled to the orthopaedic plate; a first arm (4) having a distal end (10) adapted to couple to the first drill guide (2);
a second arm (6) having a distal end coupled to the first arm (4) and a proximal end defining a pair of openings (78a, 78b) that are each adapted to releasably position a second drill guide (2a) in spaced-apart relation to the orthopaedic plate (3) such that the second guide (2a) defines a nonintersecting trajectory for the second compression screw relative to the trajectory of the first compression screw,
said guide being **characterised in that**
said first arm (4) comprises pair of beams (13a, 13b) that project outwardly from said distal end (10) in substantially parallel, spaced relation to one another, said beams (13a, 13b) including proximal ends that each define a through-bore (17a, 17b) such that said through bores (17a, 17b) are arranged in confronting, coaxial relation to one another.

2. A guide in accordance with claim 1 wherein said distal end of said first arm (4) defines a through-bore (18) that is oriented at approximately ninety degrees with respect to said through bores (17a, 17b) defined at said proximal ends of said beams (13a, 13b).

3. A guide in accordance with claim 1 wherein said first arm (4) comprises a distal end and a proximal end, a first pair of beams (30a, 30b) that project outwardly from said proximal end in substantially parallel, spaced relation to one another, and a second pair of beams (31a, 31b) that project outwardly from said distal end in substantially parallel, spaced relation to one another, wherein the free ends of said first pair and second pair of beams (30a, 30b; 31a, 31b) define confronting, coaxial through bores (33a, 33b; 35a, 35b).

4. A guide in accordance with claim 1 wherein said first arm (4) includes a threaded collet (40) projecting upwardly from said distal end.

5. A guide in accordance with claim 1 wherein said first arm (4) includes an articulating collet (60) that pivots about said distal end.

6. A guide in accordance with claim 1 wherein said second arm (6) is pivotally coupled to said first arm (4).

7. A guide in accordance with claim 1 wherein said second arm (6) includes a distal-block end (70) that defines a pair of guide bore openings and a proximal-pivot end (71) that defines a through-bore so as to be suitable for pivotal engagement with said first arm (4).

8. A guide in accordance with claim 7 wherein said guide bore openings comprise at least one of a pair of slots and a pair of through-bores arranged in substantially parallel, spaced relation to one another.

## Patentansprüche

1. Führung zur Bestimmung der Bahn einer zweiten Kompressionsschnecke relativ zu einer Stelle, an der eine erste Kompressionsschnecke anschließend in einer orthopädischen Platte (3) angeordnet sein wird, die umfasst:
eine erste Bohrführung (2), die geeignet ist, mit der orthopädischen Platte (3) gekoppelt zu werden, und die die Bahn der ersten Kompressionsschnecke definiert, wenn sie mit der orthopädischen Platte gekoppelt ist;
einen ersten Arm (4) mit einem distalen Ende (10), das geeignet ist, die mit der ersten Bohrführung (2) zu koppeln;
einen zweiten Arm (6) mit einem distalen Ende, das mit dem ersten Arm (4) gekoppelt ist, und einem proximalen Ende, das ein Paar von Öffnungen (78a, 78b) definiert, die jeweils geeignet sind, eine zweite Bohrführung (2a) in einer beabstandeten Beziehung zu der orthopädischen Platte (3) derart lösbar zu positionieren, dass die zweite Führung (2a) eine nicht schneidende Bahn für die zweite Kompressionsschnecke relativ zu der Bahn der ersten Kompressionsschnecke definiert,
wobei die Führung **dadurch gekennzeichnet ist, dass**
der erste Arm (4) ein Paar von Balken (13a, 13b) umfasst, die von dem distalen Ende (10) in einer im Wesentlichen parallelen voneinander beabstandeten Beziehung nach außen vorstehen, wobei die Balken (13a, 13b) proximale Enden umfassen, die jeweils eine Durchgangsbohrung (17a, 17b) definieren, so dass die Durchgangsbohrungen (17a, 17b) in einer gegenüberstehenden koaxialen Beziehung zueinander angeordnet sind.

2. Führung nach Anspruch 1, wobei das distale Ende des ersten Arms (4) eine Durchgangsbohrung (18) definiert, die in ungefähr 90 Grad in Bezug auf die Durchgangsbohrungen (17a, 17b), die an proximalen Enden der Balken (13a, 13b) definiert sind, orientiert ist.

3. Führung nach Anspruch 1, wobei der erste Arm (4) umfasst: ein distales Ende und ein proximales Ende, ein erstes Paar von Balken (30a, 30b), die von dem proximalen Ende in einer im Wesentlichen parallelen voneinander beabstandeten Beziehung nach außen vorstehen, und ein zweites Paar von Balken (31 a, 31 b), die von dem distalen Ende in einer im Wesentlichen parallelen voneinander beabstandeten Beziehung nach außen vorstehen, wobei die freien Enden des ersten Paars von Balken (30a, 30b; 31 a, 31 b) gegenüberstehende koaxiale Durchgangsbohrungen (33a, 33b; 35a, 35b) definieren.

4. Führung nach Anspruch 1, wobei der erste Arm (4) eine mit Gewinde versehene Klemmbuchse (40) umfasst, die von dem distalen Ende nach oben vorsteht.

5. Führung nach Anspruch 1, wobei der erste Arm (4) eine gelenkige Klemmbuchse (60) umfasst, die um das distale Ende schwenkt.

6. Führung nach Anspruch 1, wobei der zweite Arm (6) schwenkbar mit dem ersten Arm (4) gekoppelt ist.

7. Führung nach Anspruch 1, wobei der zweite Arm (6) ein distales Blockende (70), das in Paar von Führungsbohrungsöffnungen definiert, und proximales Gelenkende (71) umfasst, das eine Durchgangsbohrung definiert, so dass es für das schwenkbare Eingreifen mit dem ersten Arm (4) geeignet ist.

8. Führung gemäß Anspruch 7, wobei die Führungsbohrungsöffnungen wenigstens ein Paar Schlitze und ein Paar Durchgangslöcher umfassen, die in einer im Wesentlichen parallelen voneinander beabstandeten Beziehung angeordnet sind.

## Revendications

1. Guide déterminant la trajectoire d'une seconde vis de compression par rapport à un emplacement au niveau duquel une première vis de compression sera ultérieurement placée dans une plaque orthopédique (3) comprenant :
un premier guide de perçage (2) adapté pour être couplé avec la plaque orthopédique (3) et définissant la trajectoire de la première vis de compression lorsqu'il est couplé avec la plaque orthopédique ;
un premier bras (4) ayant une extrémité distale (10) adaptée pour être couplée avec le premier guide de perçage (2) ;
un second bras (6) ayant une extrémité distale couplée avec le premier bras (4) et une extrémité proximale définissant une paire d'ouvertures (78a, 78b) qui sont chacune adaptées pour positionner de manière amovible un second guide de perçage (2a) à distance de la plaque orthopédique (3), de telle sorte que le second guide (2a) définit , pour la seconde vis de compression, une trajectoire non sécante avec la trajectoire de la première vis de compression,
ledit guide étant **caractérisé en ce que**
ledit premier bras (4) comprend une paire de poutres (13a, 13b) qui s'étendent vers l'extérieur depuis ladite extrémité distale (10) essentiellement parallèlement et à distance l'un de l'autre, lesdites poutres (13a, 13b) comprenant des extrémités proximales qui définissent chacune un trou débouchant (17a, 17b) de telle sorte que lesdits trous débouchant (17a, 17b) sont disposés en vis-à-vis et coaxialement l'un par rapport à l'autre.

2. Guide selon la revendication 1, dans lequel ladite extrémité distale dudit premier bras (4) définit un trou débouchant (18) qui est orienté à environ quatre-vingt-dix degrés desdits trous débouchant (17a, 17b) définis au niveau desdites extrémités proximales desdites poutres (13a, 13b).

3. Guide selon la revendication 1, dans lequel ledit premier bras (4) comprend une extrémité distale et une extrémité proximale, une première paire de poutres (30a, 30b) qui s'étendent vers l'extérieur depuis ladite extrémité proximale essentiellement parallèlement et à distance l'un de l'autre, et une seconde paire de poutres (31a, 31b) qui s'étendent vers l'extérieur depuis ladite extrémité distale essentiellement parallèlement et à distance l'un de l'autre, dans lequel les extrémités libres de ladite première paire et de ladite seconde paire de poutres (30a, 30b ; 31a, 31b) définissent des trous débouchant coaxiaux se faisant face (33a, 33b ; 35a, 35b).

4. Guide selon la revendication 1, dans lequel ledit premier bras (4) comprend une douille de serrage filetée (40) dépassant vers le haut depuis ladite extrémité distale.

5. Guide selon à la revendication 1, dans lequel ledit premier bras (4) comprend une douille de serrage articulée (60) qui pivote autour de ladite extrémité distale.

6. Guide selon la revendication 1, dans lequel ledit second bras (6) est couplé de manière pivotante avec ledit premier bras (4).

7. Guide selon la revendication 1, dans lequel ledit second bras (6) comprend un bloc d'extrémité distale (70) qui définit une paire de perçages d'ouvertures de guidage et un pivot d'extrémité proximale (71) qui définit un trou débouchant de manière à être adapté à s'engager, dans une relation de pivotement, avec ledit premier bras (4).

8. Guide selon la revendication 7, dans lequel lesdits perçages d'ouvertures de guidage comprennent au moins une paire de fentes et une paire de trous débouchant disposés essentiellement parallèlement et à distance l'un de l'autre.
